# EUROPEAN PATENT APPLICATION

(11) **EP 4 219 798 A1**
(43) Date of publication of application: **02.08.2023**
(21) Application number: 21871599.3
(22) Date of filing: 24.09.2021
(51) Int. Cl.: C25D 11/26

(54) **METHOD FOR MANUFACTURING COLORED ARTICLE AND MOBILE PHONE CASE**

(30) Priority: 25.09.2020 CN 202011025357
(71) Applicant: Beijing Huayu Chuangxin Technologies Co., Ltd., Beijing 101407 (CN); Beijing Huayu Chuangxin TA-NB Science & Technology Co., Ltd., Beijing 101407 (CN)
(72) Inventor: LIU, Yuzhong, Beijing 101407 (CN)
(74) Representative: Herzog IP Patentanwalts GmbH
(86) International application number: PCT/CN2021/120331
(87) International publication number: WO 2022/063230

(57) **Abstract**

The present invention relates to a method for manufacturing a colored product, in particular a colored mobile phone shell, comprising the following steps: (1) providing a product substrate, preferably a shell substrate; and (2) surface-treating the product substrate, preferably shell substrate, by an anodic oxidation method and/or a molten salt electrochemical method, wherein the product substrate, preferably shell substrate, is made of materials selected from the group consisting of tantalum, niobium, a tantalum-niobium alloy, titanium and a titanium alloy. The present invention further relates to a colored product, in particular a colored mobile phone shell, manufactured by the above method, comprising: (1) a product substrate, preferably a shell substrate; and (2) an amorphous metal oxide layer and/or lithium-containing compound layer formed on the surface of the product substrate, preferably shell substrate, wherein the product substrate, preferably shell substrate, is made of materials selected from tantalum, niobium, a tantalum-niobium alloy, titanium and a titanium alloy.

## Description

### Technical Field

The present invention relates to a method for manufacturing a colored product, in particular a method for manufacturing a colored mobile phone shell, and to a colored product, in particular a colored mobile phone shell, manufactured by the method.

### Background Art

Colored products are often favored by people, especially mobile phones with beautiful appearances and colors can attract people's attention. With the rapid development of the mobile phone industry, the functions of the mobile phone itself are becoming more and more powerful. At the same time, the appearance of mobile phones, especially the color of mobile phone shells, has received more and more attention. In addition, since mobile phones have a certain amount of radiation, more and more attention has also been paid to manufacturing mobile phone shells with anti-radiation functions.

CN106067911A describes a method for preparing a plastic mobile phone shell, comprising the following steps: (1) proportionally mixing a plastic substrate, fluorescent powder and dyes into an injection molding machine and extruding them through a mobile phone shell mold; (2) vacuum coating after spraying primer and color paint in sequence; (3) removing the vacuum coating of the region that needs to show the color paint layer on the vacuum coating layer by laser radium carving; (4) after vacuum coating, forming an intermediate coating layer and a UV top coating layer in turn on the coating surface.

The method described in said patent has cumbersome processing steps, and the plastic mobile phone shell does not have the function of radiation protection.

CN105970270A describes a process for processing two aluminum anodic oxidized colors on an aluminum metal mobile phone shell, characterized in that it comprises the following steps:
step 1, processing an aluminum shell: using CNC to cut and process the aluminum shell;
step 2, injection molding of PPS plastics: injection molding of PPS plastics at the corresponding position of the aluminum shell made in step 1;
step 3, a first aluminum anodic oxidation coloring: after completing injection molding of PPS plastics in step 2, subjecting the whole aluminum shell to the first aluminum anodic oxidation coloring;
step 4, processing high-gloss edges: after completing the first aluminum anodic oxidation coloring in step 3, using CNC to cut and process high-gloss edges of equal width at the edge of the aluminum shell;
step 5, covering part of the high-gloss edge with photosensitive protective ink: applying photosensitive protective ink to the surface of part of the high-gloss edge, and then baking at a certain temperature for a certain period of time before exposing;
step 6, a second aluminum anodic oxidation coloring: subjecting the part of the high-gloss edge that is not covered by the photosensitive protective ink to the second aluminum anodic oxidation coloring; wherein the color of the second aluminum anodic oxidation coloring is the same as that of the first aluminum anodic oxidation coloring;
step 7, cleaning the photosensitive protective ink: using a cleaning solution to clean the photosensitive protective ink after exposure in step 5, so as to leak out the high-gloss edge;
step 8, a third aluminum anodic oxidation coloring: subjecting the high-gloss edge exposed after cleaning the exposed photosensitive protective ink in step 7 to the third aluminum anodic oxidation coloring, wherein the color of the third aluminum anodic oxidation coloring is different from that of the first aluminum anodic oxidation coloring, that is, the processing of two aluminum anodic oxidized colors on an aluminum metal mobile phone shell is completed.

The method described in this patent is also cumbersome in processing steps.

CN107567217A describes a method for preparing a housing, the method comprising: (1) providing a metal substrate, which is subjected to a hard anodic oxidation treatment to obtain a metal substrate having a metal hard anodic oxide casing; (2) forming a resin film layer on one surface of the metal substrate obtained in step (1); (3) subjecting the substrate obtained in step (2) to alkaline etching to remove the metal hard anodic oxide casing on the surface of the substrate where the resin film layer is not formed; (4) subjecting the substrate obtained in step (3) to acidic etching to remove the remaining metal in the substrate.

In said patent, the holes and etchings formed in the surface will cause the color change of the surface. In addition, the method described in said patent is also cumbersome in processing steps.

Therefore, there is still a need to find a method for manufacturing a colored product, in particular a colored mobile phone shell, which not only has a simple process and can be used to obtain rich colors, but also provides a radiation-proof function in the prepared mobile phone shell.

### Summary of the Invention

For this purpose, the present invention provides a method for manufacturing a colored product, comprising the following steps:
(1) providing a product substrate; and
(2) surface-treating the product substrate by an anodic oxidation method and/or a molten salt electrochemical method,
wherein the product substrate is made of materials selected from the group consisting of tantalum, niobium, a tantalum-niobium alloy, titanium and a titanium alloy, preferably the product substrate is made of materials selected from the group consisting of tantalum, niobium, a tantalum-niobium alloy and titanium, more preferably the product substrate is made of materials selected from the group consisting of tantalum, niobium and a tantalum-niobium alloy.

The present invention further provides a method for manufacturing a colored shell, comprising the following steps:
(1) providing a shell substrate; and
(2) surface-treating the shell substrate by an anodic oxidation method and/or a molten salt electrochemical method,
wherein the shell substrate is made of materials selected from the group consisting of tantalum, niobium, a tantalum-niobium alloy, titanium and a titanium alloy, preferably the shell substrate is made of materials selected from the group consisting of tantalum, niobium, a tantalum-niobium alloy and titanium, more preferably the shell substrate is made of materials selected from the group consisting of tantalum, niobium and a tantalum-niobium alloy.

The present invention further provides a method for manufacturing a colored mobile phone shell, comprising the following steps:
(1) providing a mobile phone shell substrate; and
(2) surface-treating the mobile phone shell substrate by an anodic oxidation method and/or a molten salt electrochemical method,
wherein the mobile phone shell substrate is made of materials selected from the group consisting of tantalum, niobium, a tantalum-niobium alloy, titanium and a titanium alloy, preferably the mobile phone shell substrate is made of materials selected from the group consisting of tantalum, niobium, a tantalum-niobium alloy and titanium, more preferably the mobile phone shell substrate is made of materials selected from the group consisting of tantalum, niobium and a tantalum-niobium alloy.

The present invention further provides a colored product manufactured by the above method, comprising:
(1) a product substrate; and
(2) an amorphous metal oxide layer and/or lithium-containing compound layer formed on the surface of the product substrate,
wherein the product substrate is made of materials selected from the group consisting of tantalum, niobium, a tantalum-niobium alloy, titanium and a titanium alloy, preferably the product substrate is made of materials selected from the group consisting of tantalum, niobium, a tantalum-niobium alloy and titanium, more preferably the product substrate is made of materials selected from the group consisting of tantalum, niobium and a tantalum-niobium alloy.

The present invention further provides a colored shell manufactured by the above method, comprising:
(1) a shell substrate; and
(2) an amorphous metal oxide layer and/or lithium-containing compound layer formed on the surface of the shell substrate,
wherein the shell substrate is made of materials selected from the group consisting of tantalum, niobium, a tantalum-niobium alloy, titanium and a titanium alloy, preferably the shell substrate is made of materials selected from the group consisting of tantalum, niobium, a tantalum-niobium alloy and titanium, more preferably the shell substrate is made of materials selected from the group consisting of tantalum, niobium and a tantalum-niobium alloy.

The present invention further provides a colored mobile phone shell manufactured by the above method, comprising:
(1) a mobile phone shell substrate; and
(2) an amorphous metal oxide layer and/or lithium-containing compound layer formed on the surface of the mobile phone shell substrate,
wherein the mobile phone shell substrate is made of materials selected from the group consisting of tantalum, niobium, a tantalum-niobium alloy, titanium and a titanium alloy, preferably the mobile phone shell substrate is made of materials selected from the group consisting of tantalum, niobium, a tantalum-niobium alloy and titanium, more preferably the mobile phone shell substrate is made of materials selected from the group consisting of tantalum, niobium and a tantalum-niobium alloy.

The method for manufacturing colored products, in particular mobile phone shells, in the present invention has one or more of the following beneficial effects: (1) colorful products, in particular mobile phone shells, can be obtained; (2) the manufactured colored mobile phone shells have the effect of reducing mobile phone radiation; (3) the manufacturing process is simple, and (4) it is easy to repair the color and replace the color.

### Description of Drawings

Figure 1 shows colored tantalum foils prepared under different process conditions according to embodiments of the present invention.
Figure 2 shows colored tantalum mobile phone shells prepared under different process conditions according to embodiments of the present invention.
Figure 3 shows colored niobium foils prepared under different process conditions according to embodiments of the present invention.
Figure 4 shows colored tantalum-niobium alloy foils prepared under different process conditions according to embodiments of the present invention.
Figure 5 shows colored titanium foils prepared under different process conditions according to embodiments of the present invention.
Figure 6 shows colored titanium mobile phone shells prepared in molten lithium nitrate under different process conditions according to embodiments of the present invention.
Figure 7 shows colored titanium mobile phone shells prepared in molten lithium nitrate under different process conditions according to another embodiments of the present invention.
Figure 8 shows off-white and white tantalum, niobium and tantalum-niobium alloy foils prepared in molten lithium nitrate according to embodiments of the present invention.
Figure 9 shows titanium mobile phone shells with colorful patterns prepared according to embodiments of the present invention.
Figure 10 shows tantalum mobile phone shells with colorful patterns prepared in 0.05 wt% H₃PO₄ aqueous solution according to embodiments of the present invention.
Figure 11 shows niobium mobile phone shells with colorful patterns prepared in 0.05 wt% H₃PO₄ aqueous solution according to embodiments of the present invention.

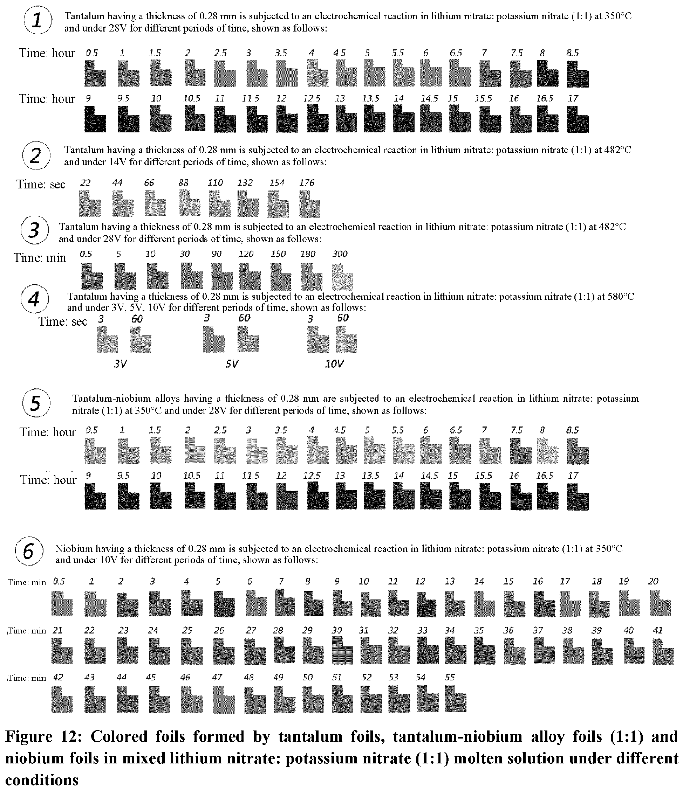

Figure 12 shows colored tantalum, niobium, and tantalum-niobium alloy foils prepared in lithium nitrate: potassium nitrate (1: 1 weight ratio) according to embodiments of the present invention.

### Specific embodiments

The method for manufacturing colored products (such as mobile phone shells) of the present invention can be carried out by using an anodic oxidation method and/or a molten salt electrochemical method. In particular, for tantalum, niobium and a tantalum-niobium alloy, an anodic oxidation method can be used alone, or a combination of anodic oxidation method and molten salt electrochemical method can be used to prepare colored products, including colored mobile phone shells, while for titanium and a titanium alloy, it is preferred to use an anodic oxidation method, and more preferably a molten salt anodic oxidation method to prepare colored products, including colored mobile phone shells.

### Anodic Oxidation Method

The anodic oxidation is carried out in an oxygen-containing electrolyte solution with a product such as a mobile phone shell made of tantalum, niobium, a tantalum-niobium alloy, titanium and a titanium alloy as the anode and with nickel or stainless steel or other metals stable to the oxygen-containing electrolyte solution as the cathode by applying a suitable anodic voltage and maintaining the voltage constant for a suitable period of time at a suitable temperature.

In one embodiment, the method for manufacturing a colored product in the present invention comprises the steps of:
(1) providing a product substrate; and
(2) surface-treating the product substrate by an anodic oxidation method,
wherein the product substrate is made of materials selected from the group consisting of tantalum, niobium, a tantalum-niobium alloy, titanium and a titanium alloy, preferably the product substrate is made of materials selected from the group consisting of tantalum, niobium, a tantalum-niobium alloy and titanium, more preferably the product substrate is made of materials selected from the group consisting of tantalum, niobium and a tantalum-niobium alloy, further preferably the product substrate is made of materials selected from the group consisting of titanium and a titanium alloy.

In one embodiment, the method for manufacturing a colored shell in the present invention comprises the steps of:
(1) providing a shell substrate; and
(2) surface-treating the shell substrate by an anodic oxidation method,
wherein the shell substrate is made of materials selected from the group consisting of tantalum, niobium, a tantalum-niobium alloy, titanium and a titanium alloy, preferably the shell substrate is made of materials selected from the group consisting of tantalum, niobium, a tantalum-niobium alloy and titanium, more preferably the shell substrate is made of materials selected from the group consisting of tantalum, niobium and a tantalum-niobium alloy, further preferably the shell substrate is made of materials selected from the group consisting of titanium and a titanium alloy.

In one embodiment, the method for manufacturing a colored mobile phone shell in the present invention comprises the steps of:
(1) providing a mobile phone shell substrate; and
(2) surface-treating the mobile phone shell substrate by an anodic oxidation method,
wherein the mobile phone shell substrate is made of materials selected from the group consisting of tantalum, niobium, a tantalum-niobium alloy, titanium and a titanium alloy, preferably the mobile phone shell substrate is made of materials selected from the group consisting of tantalum, niobium, a tantalum-niobium alloy and titanium, more preferably the mobile phone shell substrate is made of materials selected from the group consisting of tantalum, niobium and a tantalum-niobium alloy, further preferably the mobile phone shell substrate is made of materials selected from the group consisting of titanium and a titanium alloy.

In a preferred embodiment, said substrate, such as shell substrate and mobile phone shell substrate, is made of tantalum.

In a preferred embodiment, said substrate, such as shell substrate and mobile phone shell substrate, is made of niobium.

In a preferred embodiment, said substrate, such as shell substrate and mobile phone shell substrate, is made of a tantalum-niobium alloy.

In a preferred embodiment, said substrate, such as shell substrate and mobile phone shell substrate, is made of titanium.

In a preferred embodiment, said substrate, such as shell substrate and mobile phone shell substrate, is made of a titanium alloy.

Preferably, the anodic oxidation method is carried out under the following conditions: the temperature of the electrolyte solution is 20°C-600°C, the anode voltage is 1V-800V, the constant voltage time is 0.01-2 hours, and the boosting current density is 1-200mA/cm², so as to form an amorphous metal oxide layer on the surface of substrates such as shell substrates and mobile phone shell substrates.

The electrolyte solution comprises an aqueous solution, a mixture of an aqueous solution and an organic compound, and a non-aqueous solution.

In one embodiment, the aqueous solution comprises an aqueous solution of acid, base or salt, preferably an aqueous solution of phosphoric acid. Preferably, the aqueous solution of phosphoric acid has a concentration of 0.01-1 wt%, preferably 0.01-0.1 wt%, more preferably 0.01-0.05 wt%.

In one embodiment, in a mixture of an aqueous solution and an organic compound, the aqueous solution comprises an aqueous solution of acid, base, or salt, and the organic compound comprises an alcoholic organic compound, such as ethanol, ethylene glycol, n-butanol, or any combination thereof.

In a preferred embodiment, the mixture of an aqueous solution and an organic compound comprises a mixture of a phosphoric acid aqueous solution having a concentration of 0.01-0.05 wt% and an alcohol, such as ethylene glycol, in a volume ratio of 1:1 to 1:3, preferably 1: 1 to 2:1, more preferably 2: 1. Preferably, the mixture of an aqueous solution and an organic compound comprises a mixture of a phosphoric acid aqueous solution having a concentration of 0.05 wt% and ethylene glycol in a weight ratio of 2:1.

Preferably, the non-aqueous solution comprises anhydrous concentrated sulfuric acid, molten salt, and a mixture of molten salt and alkaline substance. Preferably, the molten salt comprises molten lithium nitrate, molten sodium nitrate, molten potassium nitrate and any combination thereof. Preferably, the alkaline substance comprises lithium hydroxide, sodium hydroxide, potassium hydroxide and any combination thereof.

In one embodiment, the anodic oxidation method can be carried out by placing a substrate such as a shell substrate or a mobile phone shell substrate in an aqueous electrolyte solution or a mixed solution of an aqueous electrolyte solution and an organic compound. Preferably, the solution temperature is 1-99°C, preferably 10-95°C, more preferably from room temperature to 90 °C, the anode voltage is 5-300V, the constant voltage time is 0.5-1.5 hours, and the boosting current density is 5-25 mA/cm².

The temperature of the solution is preferably controlled at room temperature to 95°C. If the temperature is too high, the moisture will evaporate too fast. Higher solution temperature, higher anode voltage, and longer constant voltage time can easily lead to the crystallization of the amorphous metal oxide layer, the crystallized metal oxide layer no longer refracts or transmits light, and therefore does not change color (generally gray or off-white) with the change in film thickness.

In one embodiment, the method for manufacturing a colored mobile phone shell comprises the following steps:
(1) providing a mobile phone shell substrate; and
(2) surface-treating the mobile phone shell substrate by an anodic oxidation method in an aqueous solution,
wherein the mobile phone shell substrate is made of tantalum.

The aqueous solution is preferably an aqueous phosphoric acid solution, more preferably an aqueous phosphoric acid solution having a concentration of 0.01-0.05 wt%.

In a preferred embodiment, a tantalum mobile phone shell with a thickness of 0.01-0.3mm is prepared by applying an anode voltage of 28-32V to the tantalum mobile phone shell placed in a 0.01-0.05 wt% aqueous H₃PO₄ solution preferably at room temperature (25°C) and maintaining the voltage constant for 0.5-1.5 hours using an anodic oxidation method, to obtain a blue mobile phone shell.

In a preferred embodiment, a tantalum mobile phone shell with a thickness of 0.01-0.3mm is prepared by applying an anode voltage of 72-84V to the tantalum mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.

In a preferred embodiment, a tantalum mobile phone shell with a thickness of 0.01-0.3mm is prepared by applying an anode voltage of 91-103V to the tantalum mobile phone shell placed in a 0.01-0.05 wt% aqueous H₃PO₄ solution preferably at room temperature (25°C) and maintaining the voltage constant for 0.5-1.5 hours using an anodic oxidation method, to obtain a blue purple mobile phone shell.

In a preferred embodiment, a tantalum mobile phone shell with a thickness of 0.01-0.3mm is prepared by applying an anode voltage of 150-160V to the tantalum mobile phone shell placed in a 0.01-0.05 wt% aqueous H₃PO₄ solution preferably at room temperature (25°C) and maintaining the voltage constant for 0.5-1.5 hours using an anodic oxidation method, to obtain a pink mobile phone shell.

In a preferred embodiment, a tantalum mobile phone shell with a thickness of 0.01-0.3mm is prepared by applying an anode voltage of 203-210V to the tantalum mobile phone shell placed in a 0.01-0.05 wt% aqueous H₃PO₄ solution preferably at room temperature (25°C) and maintaining the voltage constant for 0.5-1.5 hours using an anodic oxidation method, to obtain a green mobile phone shell.

In another embodiment, the method for manufacturing a colored mobile phone shell comprises the following steps:
(1) providing a mobile phone shell substrate; and
(2) surface-treating the mobile phone shell substrate by an anodic oxidation method in an aqueous solution,
wherein the mobile phone shell substrate is made of niobium.

The aqueous solution is preferably an aqueous phosphoric acid solution, more preferably an aqueous phosphoric acid solution having a concentration of 0.01-0.05 wt%.

In a preferred embodiment, a niobium mobile phone shell with a thickness of 0.01-0.3mm is prepared by applying an anode voltage of 21-29V to the niobium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a blue mobile phone shell.

In a preferred embodiment, a niobium mobile phone shell with a thickness of 0.01-0.3mm is prepared by applying an anode voltage of 31-38V to the niobium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a silver-white mobile phone shell.

In a preferred embodiment, a niobium mobile phone shell with a thickness of 0.01-0.3mm is prepared by applying an anode voltage of 40-48V to the niobium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.

In a preferred embodiment, a niobium mobile phone shell with a thickness of 0.01-0.3mm is prepared by applying an anode voltage of 71-78V to the niobium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a green mobile phone shell.

In a preferred embodiment, a niobium mobile phone shell with a thickness of 0.01-0.3mm is prepared by applying an anode voltage of 105-113V to the niobium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a purplish red mobile phone shell.

In yet another embodiment, the method for manufacturing a colored mobile phone shell comprises the following steps:
(1) providing a mobile phone shell substrate; and
(2) surface-treating the mobile phone shell substrate by an anodic oxidation method in an aqueous solution,
wherein the mobile phone shell substrate is made of a tantalum-niobium alloy.

The aqueous solution is preferably an aqueous phosphoric acid solution, more preferably an aqueous phosphoric acid solution having a concentration of 0.01-0.05 wt%.

In a preferred embodiment, a tantalum-niobium alloy mobile phone shell with a thickness of 0.01-0.3mm is prepared by applying an anode voltage of 8-18V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a chocolate-colored mobile phone shell.

In a preferred embodiment, a tantalum-niobium alloy mobile phone shell with a thickness of 0.01-0.3mm is prepared by applying an anode voltage of 27-28V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a sky-blue mobile phone shell.

In a preferred embodiment, a tantalum-niobium alloy mobile phone shell with a thickness of 0.01-0.3mm is prepared by applying an anode voltage of 38-46V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a silver-gray mobile phone shell.

In a preferred embodiment, a tantalum-niobium alloy mobile phone shell with a thickness of 0.01-0.3mm is prepared by applying an anode voltage of 97-102V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a marine green mobile phone shell.

In a preferred embodiment, a tantalum-niobium alloy mobile phone shell with a thickness of 0.01-0.3mm is prepared by applying an anode voltage of 138-145V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a violet mobile phone shell.

In a preferred embodiment, a tantalum-niobium alloy mobile phone shell with a thickness of 0.01-0.3mm is prepared by applying an anode voltage of 246-251V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a dark brown mobile phone shell.

In one embodiment, the method for manufacturing a colored mobile phone shell comprises the following steps:
(1) providing a mobile phone shell substrate; and
(2) surface-treating the mobile phone shell substrate by an anodic oxidation method in an aqueous solution,
wherein the mobile phone shell substrate is made of titanium.

The aqueous solution is preferably an aqueous phosphoric acid solution, more preferably an aqueous phosphoric acid solution having a concentration of 0.01-0.05 wt%.

In a preferred embodiment, a titanium mobile phone shell with a thickness of 0.01-0.3mm is prepared by applying an anode voltage of 44-54V to the titanium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a white-green mobile phone shell.

In a preferred embodiment, a titanium mobile phone shell with a thickness of 0.01-0.3mm is prepared by applying an anode voltage of 72-84V to the titanium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.

In a preferred embodiment, a titanium mobile phone shell with a thickness of 0.01-0.3mm is prepared by applying an anode voltage of 92-109V to the titanium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a purplish red mobile phone shell.

In a preferred embodiment, a titanium mobile phone shell with a thickness of 0.01-0.3mm is prepared by applying an anode voltage of 121-133V to the titanium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a green mobile phone shell.

In a preferred embodiment, a titanium mobile phone shell with a thickness of 0.01-0.3mm is prepared by applying an anode voltage of 159-180V to the titanium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a brown-gray mobile phone shell.

The anodic oxidation method can also be carried out by placing the shell substrate in a non-aqueous electrolyte solution. The temperature of the electrolyte solution is from the melting point of the substance of the electrolyte solution to 500°C, the anode voltage is 3V-66V, and the constant voltage time is 0.1-80 minutes. Preferably, the temperature of the electrolyte solution is 250-380°C, the anode voltage is 10-40V, and the constant voltage time is 3 seconds-40 minutes.

In one embodiment, the method for manufacturing a colored shell, in particular a mobile phone shell, comprises the following steps:
(1) providing a shell substrate; and
(2) surface-treating the shell substrate by an anodic oxidation method in a non-aqueous solution,
wherein the shell substrate is made of materials selected from the group consisting of tantalum, niobium, a tantalum-niobium alloy, titanium and a titanium alloy.

The oxide film formed in the non-aqueous solution is porous, and the thickness thereof during the formation of the oxide film, that is, the color, is not only related to temperature and anode voltage, but also related to the constant voltage time, while for the anodic oxidation in an aqueous solution, it has nothing to do with the constant voltage time.

The non-aqueous solution comprises anhydrous concentrated sulfuric acid, molten salt, and a mixture of molten salt and alkaline substance. Preferably, the molten salt comprises molten lithium nitrate, molten sodium nitrate, molten potassium nitrate and any combination thereof. Preferably, the alkaline substance comprises lithium hydroxide, sodium hydroxide, potassium hydroxide and any combination thereof.

The non-aqueous solution is preferably a molten salt, including molten potassium nitrate, molten sodium nitrate, molten lithium nitrate and any combination thereof. Preferably, the molten salt is molten lithium nitrate.

In one embodiment, the method for manufacturing a colored shell, in particular a mobile phone shell, comprises the following steps:
(1) providing a shell substrate; and
(2) surface-treating the shell substrate by an anodic oxidation method in a non-aqueous solution,
wherein the shell substrate is made of titanium.

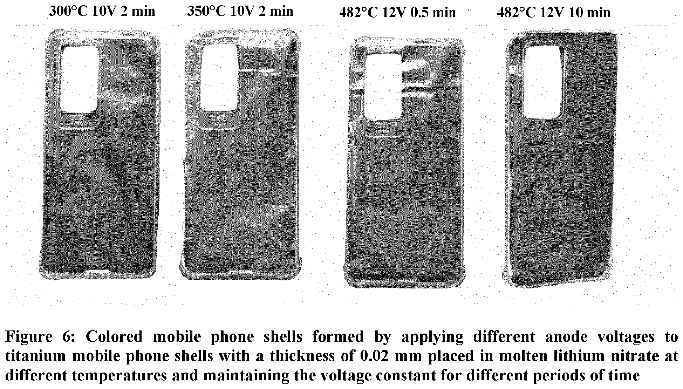

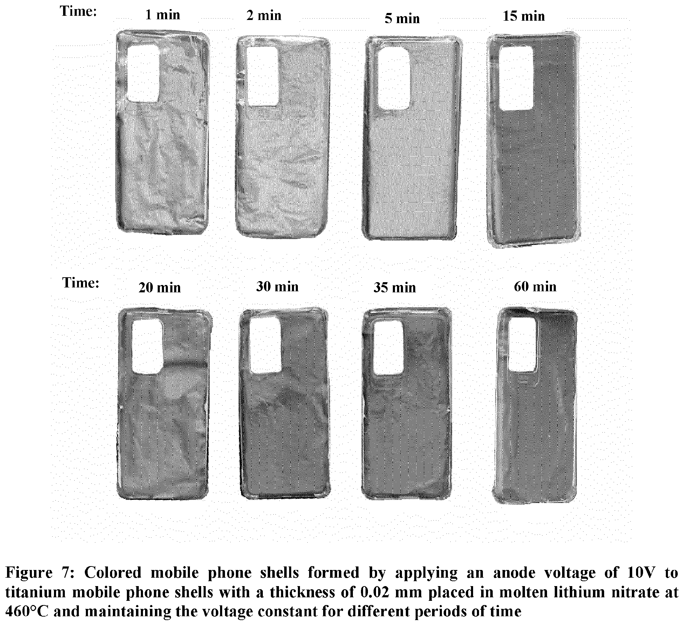

As shown in Figures 6 and 7, in a preferred embodiment, the shell substrate made of titanium is placed in lithium nitrate at 460°C and subjected to an anodic oxidation method treatment at a voltage of 10V for 5 minutes to obtain a golden yellow shell.

In a preferred embodiment, the shell substrate made of titanium is placed in lithium nitrate at 482°C and subjected to an anodic oxidation method treatment at a voltage of 12V for 10 minutes to obtain a purple shell.

In a preferred embodiment, the shell substrate made of titanium is placed in lithium nitrate at 460°C and subjected to an anodic oxidation method treatment at a voltage of 10V for 60 minutes to obtain a royal blue shell.

In a preferred embodiment, the shell substrate made of titanium is placed in lithium nitrate at 300-350°C and subjected to an anodic oxidation method treatment at a voltage of 10V for 2 minutes to obtain a sky-blue shell.

In a preferred embodiment, the shell substrate made of titanium is placed in lithium nitrate at 482°C and subjected to an anodic oxidation method treatment at a voltage of 12V for 10 minutes to obtain a purple shell.

In one embodiment, the present invention provides a method for manufacturing a colored product with multiple colored patterns, in particular a colored mobile phone shell, comprising the following steps:
(1) forming a colored pattern on the product substrate by an anodic oxidation method and/or a molten salt electrochemical method according to a predetermined pattern; and
(2) sheltering the formed colored pattern, and then forming another colored pattern by an anodic oxidation method and/or a molten salt electrochemical method.

In a preferred embodiment, in the method for manufacturing a colored product with multiple colored patterns, in particular a colored mobile phone shell, it is feasible to first protect a part of the mobile phone shell with a specific patterned adhesive tape, then anodic oxidize the mobile phone shell to form a colored pattern, peel off the tape and protect the colored pattern formed on the mobile phone shell with another adhesive tape, and further anodic oxidize the mobile phone shell to obtain another colored pattern. Certainly, multiple adhesive tapes can be used to protect the mobile phone shell during anodic oxidation in order to obtain a mobile phone shell with multiple colors, such as three or more colors. The adhesive tape used is usually waterproof and resistant to the employed anodic oxidation conditions.

In a preferred embodiment, the method for manufacturing a colored product with multiple colored patterns, in particular a colored mobile phone shell, comprises:
(1) determining the metal material and specific pattern on the mobile phone shell;
(2) determining the color used for each pattern on the mobile phone shell;
(3) determining the order of surface treatment used to realize the color of each pattern on the mobile phone shell, wherein the order should conform to the principle that the thickness of the oxide film is gradually reduced, that is, the film thickness of the corresponding pattern for the color which is first subjected to surface treatment (or anodic oxidation treatment) should be greater than the thickness of the film for which the color is later applied; and
(4) using a waterproof and acid-proof adhesive tape to protect the part other than the first anodic oxidized pattern, and tearing it off after the first anodic oxidation treatment is completed, and then using this tape protection method to carry out the anodic oxidation treatment of the next pattern, until finally all patterns are processed.

The waterproof tape should also be resistant to the anodic oxidation conditions employed in terms of such as temperature, acid resistance.

### Molten Salt Electrochemical Method

In one embodiment, the method for manufacturing a colored shell, in particular a mobile phone shell, comprises the following steps:
(1) providing a shell substrate; and
(2) surface-treating the shell substrate by a molten salt electrochemical method,
wherein the shell substrate is made of materials selected from the group consisting of tantalum, niobium and a tantalum-niobium alloy.

Preferably, the molten salt electrochemical method is a molten lithium salt electrochemical method.

Preferably, the molten salt electrochemical method comprises placing the shell substrate in an oxygen-containing inorganic lithium salt, a mixed melt of oxygen-containing inorganic lithium salt and lithium hydroxide, a mixed molten solution of salt and lithium hydroxide, or a mixed molten solution of lithium salt and oxygen-containing salt at 200°C-650°C, applying an anode voltage of 1-66V and maintaining the voltage constant for 3 seconds-60 hours, for example 0.1-90 minutes, wherein the boosting current density is 1-1000 mA/cm², to obtain a white or off-white shell. Preferably, the ultrasonic generator is set in said mixed melt or in said mixed molten solution.

Preferably, the temperature of the mixed melt or the mixed molten solution is 250°C-520°C, the applied anode voltage is 5-25V, and the boosting current density is 5-20 mA/cm².

In a preferred embodiment, the oxygen-containing inorganic lithium salt is LiNO₃.

In a preferred embodiment, the mixed molten solution of lithium salt and oxygen-containing salt is a mixed molten solution of LiNO₃ and KNO₃.

In a preferred embodiment, the mixed molten solution of lithium salt and oxygen-containing salt is a mixed molten solution of LiNO₃ and KNO₃ in a weight ratio of 1:2 to 2:1, preferably 1: 1.

For the mixed melt or the mixed molten solution, for example nitrates such as LiNO₃, NaNO₃ and KNO₃, the thickness of the amorphous metal oxide film (layer) generated at higher temperatures increases with the increase of anodic oxidation time. After reaching a certain thickness, the amorphous metal oxide film will electrochemically react with nitrate to form a white or off-white lithium tantalate film, lithium niobate film, mixed lithium tantalate and lithium niobate film, especially at higher temperatures and higher anode voltages.

In one embodiment, the method for manufacturing a colored shell, in particular a mobile phone shell, comprises the following steps:
(1) providing a shell substrate; and
(2) surface-treating the shell substrate by a molten salt electrochemical method,
wherein the shell substrate is made of tantalum.

In one embodiment, the shell substrate made of tantalum is placed in lithium nitrate at 440°C-650°C and subjected to a constant voltage of 5-50V for 3 seconds-5 hours to obtain a white shell.

In a preferred embodiment, the shell substrate made of tantalum is placed in lithium nitrate at 570°C-585°C and subjected to a constant voltage of 8-50V for 2 minutes-5 hours to obtain a white shell.

In one embodiment, the shell substrate made of niobium is placed in lithium nitrate: potassium nitrate (a weight ratio of 1:2 to 2:1, preferably a weight ratio of 1:1) at 380°C-550°C and subjected to a constant voltage of 5-48V for 2 seconds-2 hours to obtain a white shell.

In a preferred embodiment, the shell substrate made of niobium is placed in lithium nitrate: potassium nitrate (a weight ratio of 1:2 to 2:1, preferably a weight ratio of 1:1) at 450°C-480°C and subjected to a constant voltage of 5-28V for 2-30 minutes to obtain a white shell.

In one embodiment, the method for manufacturing a colored shell, in particular a mobile phone shell, comprises the following steps:
(1) providing a shell substrate; and
(2) surface-treating the shell substrate by an anodic oxidation method and a molten salt electrochemical method,
wherein the shell substrate is made of materials selected from the group consisting of tantalum, niobium and a tantalum-niobium alloy.

In a preferred embodiment, the shell substrate made of a tantalum-niobium alloy is placed in a 0.01-0.05 wt% aqueous H₃PO₄ solution and subjected to a constant voltage of 135-145V for 1-2 hours, and then placed in lithium nitrate: potassium nitrate (a weight ratio of 1:2 to 2:1, preferably a weight ratio of 1:1) at 460-585°C and subjected to a constant voltage of 25-32V for 1-3 hours to obtain a white shell.

It is also possible to form white or other colored combination patterns on the mobile phone shell through the combination of anodic oxidation method and molten salt electrochemical method. For example, on the mobile phone shell made of tantalum, niobium, or a tantalum-niobium alloy, the method of electrochemical reaction can be carried out by applying an appropriate anode voltage in the molten oxygen-containing lithium salt, such as lithium nitrate, or the mixed molten solution of lithium nitrate with potassium nitrate or sodium nitrate, etc., and the corresponding white lithium tantalate film, or lithium niobate film, or the mixed film of lithium tantalate and lithium niobate can be generated by the electrochemical reaction. If a white pattern is desired while the contrasting base color is a colored anodic oxidized film, then the white film outside the pattern can be removed by mechanical polishing or by HF acid etching until the metallic tantalum, niobium, or tantalum-niobium alloy is exposed, which is then anodic oxidized in dilute aqueous solution or molten salt to produce the designed colored anodic oxidized film.

The colored product manufactured according to the method of the present invention comprises:
(1) a product substrate; and
(2) an amorphous metal oxide layer and/or a lithium-containing compound layer, preferably an amorphous metal oxide layer, formed on the surface of the shell substrate,
wherein the product substrate is made of materials selected from the group consisting of tantalum, niobium, a tantalum-niobium alloy, titanium and a titanium alloy, preferably the product substrate is made of materials selected from the group consisting of tantalum, niobium, a tantalum-niobium alloy and titanium, more preferably the product substrate is made of materials selected from the group consisting of tantalum, niobium and a tantalum-niobium alloy.

The colored shell manufactured according to the method of the present invention comprises:
(1) a product shell substrate; and
(2) an amorphous metal oxide layer and/or a lithium-containing compound layer, preferably an amorphous metal oxide layer, formed on the surface of the shell substrate,
wherein the shell substrate is made of materials selected from the group consisting of tantalum, niobium, a tantalum-niobium alloy, titanium and a titanium alloy, preferably the shell substrate is made of materials selected from the group consisting of tantalum, niobium, a tantalum-niobium alloy and titanium, more preferably the shell substrate is made of materials selected from the group consisting of tantalum, niobium and a tantalum-niobium alloy.

The colored mobile phase shell manufactured according to the method of the present invention comprises:
(1) a product mobile phone shell substrate; and
(2) an amorphous metal oxide layer and/or a lithium-containing compound layer, preferably an amorphous metal oxide layer, formed on the surface of the mobile phase shell substrate,
wherein the mobile phone shell substrate is made of materials selected from the group consisting of tantalum, niobium, a tantalum-niobium alloy, titanium and a titanium alloy, preferably the mobile phone shell substrate is made of materials selected from the group consisting of tantalum, niobium, a tantalum-niobium alloy and titanium, more preferably the mobile phone shell substrate is made of materials selected from the group consisting of tantalum, niobium and a tantalum-niobium alloy.

In one embodiment, the amorphous metal oxide comprises amorphous tantalum pentoxide, amorphous niobium pentoxide, amorphous titanium dioxide, and mixtures thereof.

In a preferred embodiment, the lithium-containing compound comprises lithium tantalate, lithium niobate, and mixtures thereof.

In a preferred embodiment, the shell substrate has a thickness of 0.01-0.5mm, preferably 0.02-0.3mm.

The shell substrate of the present invention can be produced by techniques known in the art, such as cutting, casting, calendering, etc., without any particular limitation.

In the context of the present invention, the mobile phone shell comprises a mobile phone body outer shell, a mobile phone decorative outer shell, a mobile phone protective outer shell, and the like.

In the context of the present invention, the colored product comprises shells, such as mobile phone shells and computer shells, etc.; accessories, such as rings, bracelets, necklaces, and watch straps; pendants such as Buddha statues, human figures, and animal images.

In one embodiment, a transparent plastic shell can be added outside the mobile phone shell of the present invention to protect the mobile phone from being damaged when bumped accidentally. Embedding the mobile phone shell of the present invention in the transparent plastic shell can achieve the purposes of anti-scratch and anti-deformation, and at the same time, the transparent plastic shell makes it possible to appreciate the bright colors of the colored mobile phone shell. In addition, the mobile phone decorative outer shell or mobile phone protective outer shell embedded in the transparent plastic shell can be easily replaced with shells having other colors.

In another embodiment, the mobile phone shell of the present invention, such as a mobile phone decorative outer shell or a mobile phone protective outer shell, can also be integrated with a plastic transparent shell, for example, by placing the metal mobile phone shell of the present invention in a transparent plastic shell, so as to better ensure that the metal mobile phone shell will not be scratched, worn or deformed due to careless operation and other reasons when the plastic shell of the mobile phone is taken off.

In the context of the present invention, "color" should be understood to include, but not limited to, red, green, blue, yellow, purple, golden yellow, white, silvery white, gray and the like.

Unless otherwise indicated, all percentages of the present invention are calculated by weight.

The present invention can be more easily understood by those skilled in the art in accordance with the following preferred embodiments, but said preferred embodiments are not intended to limit the scope of the present invention.
1. A method for manufacturing a colored product, comprising the following steps:
   (1) providing a product substrate; and
   (2) surface-treating the product substrate by an anodic oxidation method and/or a molten salt electrochemical method,
   wherein the product substrate is made of materials selected from the group consisting of tantalum, niobium, a tantalum-niobium alloy, titanium and a titanium alloy.
2. The method according to embodiment 1, comprising following steps:
   (1) providing a shell substrate; and
   (2) surface-treating the shell substrate by an anodic oxidation method and/or a molten salt electrochemical method,
   wherein the shell substrate is made of materials selected from the group consisting of tantalum, niobium, a tantalum-niobium alloy, titanium and a titanium alloy.
3. The method according to embodiment 2, comprising following steps:
   (1) providing a mobile phone shell substrate; and
   (2) surface-treating the mobile phone shell substrate by an anodic oxidation method and/or a molten salt electrochemical method,
   wherein the mobile phone shell substrate is made of materials selected from the group consisting of tantalum, niobium, a tantalum-niobium alloy, titanium and a titanium alloy.
4. The method according to any one of embodiments 1-3, wherein step (2) comprises:
   - forming a colored pattern on the substrate by an anodic oxidation method and/or a molten salt electrochemical method according to a predetermined pattern; and
   - sheltering the formed colored pattern, and then forming another colored pattern by an anodic oxidation method and/or a molten salt electrochemical method.
5. The method according to any one of embodiments 1-3, wherein the anodic oxidation method is carried out under the following conditions: the temperature of the electrolyte solution is 20°C-600°C, the anode voltage is 1V-800V, the constant voltage time is 3 seconds-5 hours, preferably 30 seconds-1.5 hours, and the boosting current density is 1-200mA/cm², so as to form an amorphous metal oxide layer and/or a lithium-containing compound layer on the surface of substrates.
6. The method according to embodiment 5, wherein the electrolyte solution comprises an aqueous solution, a mixture of an aqueous solution and an organic compound, and a non-aqueous solution.
7. The method according to embodiment 6, wherein the aqueous solution comprises an aqueous solution of acid, base or salt, preferably an aqueous solution of phosphoric acid, and the aqueous solution of phosphoric acid has a concentration of 0.01-1 wt%, preferably 0.01-0.05 wt%.
8. The method according to embodiment 6, wherein in a mixture of an aqueous solution and an organic compound, the aqueous solution comprises an aqueous solution of acid, base, or salt, and the organic compound comprises an alcohol, such as ethanol, ethylene glycol, n-butanol, or any combination thereof.
9. The method according to embodiment 6, wherein the mixture of an aqueous solution and an organic compound comprises a mixture of a phosphoric acid aqueous solution having a concentration of 0.01-0.05 wt% and ethylene glycol in a weight ratio of 1:1 to 3:1, more preferably 1: 1 to 2:1.
10. The method according to embodiment 6, wherein the non-aqueous solution comprises anhydrous concentrated sulfuric acid, molten salt, and a mixture of molten salt and alkaline substance.
11. The method according to embodiment 10, wherein the molten salt comprises molten lithium nitrate, molten sodium nitrate, molten potassium nitrate and any combination thereof.
12. The method according to embodiment 10, wherein the alkaline substance comprises lithium hydroxide, sodium hydroxide, potassium hydroxide and any combination thereof.
13. The method according to embodiment 6, wherein when the electrolyte solution is an aqueous solution or a mixture of an aqueous solution and an organic compound, the anodic oxidation method is carried out under the following conditions: the temperature of the electrolyte solution is 1-99°C, and the anode voltage is 5V-600V, and the constant voltage time is within 90 minutes; preferably, the temperature of the electrolyte solution is from room temperature to 95°C, the anode voltage is 10-200V, and the constant voltage time is 30-60 minutes.
14. The method according to embodiment 13, wherein a tantalum mobile phone shell with a thickness of 0.01-0.3mm is prepared by applying an anode voltage of 72-84V to the tantalum mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.
15. The method according to embodiment 13, wherein a niobium mobile phone shell with a thickness of 0.01-0.3mm is prepared by applying an anode voltage of 40-48V to the niobium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.
16. The method according to embodiment 13, wherein a tantalum-niobium alloy mobile phone shell with a thickness of 0.01-0.3mm is prepared by applying an anode voltage of 27-28V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a sky-blue mobile phone shell.
17. The method according to embodiment 13, wherein a titanium mobile phone shell with a thickness of 0.01-0.3mm is prepared by applying an anode voltage of 72-84V to the titanium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.
18. The method according to embodiment 6, wherein when the electrolyte solution is a non-aqueous solution, the anodic oxidation method is carried out under the following conditions: the temperature of the electrolyte solution is from the melting point of the substance of the electrolyte solution to 500°C, the anode voltage is 3V-66V, and the constant voltage time is within 60 minutes; preferably, the temperature of the electrolyte solution is 250-350°C, the anode voltage is 5-40V, and the constant voltage time is 3-30 minutes.
19. The method according to embodiment 18, wherein the shell substrate made of titanium is placed in lithium nitrate at 460°C and subjected to an anodic oxidation method treatment at a voltage of 10V for 5 minutes to obtain a golden yellow shell.
20. The method according to any one of embodiments 1-3, wherein the molten salt electrochemical method comprises placing the substrate in an oxygen-containing inorganic lithium salt, a mixed melt of oxygen-containing inorganic lithium salt and lithium hydroxide, a mixed molten solution of salt and lithium hydroxide, or a mixed molten solution of lithium salt and oxygen-containing salt at 200°C-650°C, applying an anode voltage of 1-66V and maintaining the voltage constant for 0.01-60 hours, wherein the boosting current density is 1-1000 mA/cm², to obtain a white or off-white mobile phone shell.
21. The method according to embodiment 20, wherein the temperature of the mixed melt or the mixed molten solution is 250°C-520°C, the applied anode voltage is 5-25V, and the boosting current density is 5-20 mA/cm².
22. The method according to embodiment 20, wherein the oxygen-containing inorganic lithium salt is LiNO₃.
23. The method according to embodiment 20, wherein the shell substrate made of tantalum is placed in lithium nitrate at 440°C-650°C and subjected to a constant voltage of 8-50V for 2 minutes-5 hours to obtain a white shell.
24. The method according to embodiment 20, wherein the shell substrate made of niobium is placed in a lithium nitrate/potassium nitrate mixture in a weight ratio of 1:2 to 2:1, preferably a weight ratio of 1:1 at 380°C-550°C and subjected to a constant voltage of 5-28V for 2-30 minutes to obtain a white shell.
25. The method according to embodiment 2 or 3, comprising the following steps:
   (1) providing a shell substrate; and
   (2) surface-treating the shell substrate by an anodic oxidation method and a molten salt electrochemical method,
   wherein the shell substrate is made of materials selected from the group consisting of tantalum, niobium and a tantalum-niobium alloy.
26. The method according to embodiment 25, wherein the shell substrate made of a tantalum-niobium alloy is placed in a 0.01-0.05 wt% aqueous H₃PO₄ solution and subjected to a constant voltage of 135-145V for 1-2 hours, and then placed in a lithium nitrate/potassium nitrate mixture in a weight ratio of 1:2 to 2:1, preferably a weight ratio of 1:1 at 400-585°C and subjected to a constant voltage of 25-32V for 1-3 hours to obtain a white shell.
27. A colored product manufactured according to the method of any one of embodiments 1-26, comprising:
   (1) a product substrate; and
   (2) an amorphous metal oxide layer and/or a lithium-containing compound layer, preferably an amorphous metal oxide layer, formed on the surface of the product substrate.
   wherein the product substrate is made of materials selected from the group consisting of tantalum, niobium, a tantalum-niobium alloy, titanium and a titanium alloy, preferably the product substrate is made of materials selected from the group consisting of tantalum, niobium, a tantalum-niobium alloy and titanium, more preferably the product substrate is made of materials selected from the group consisting of tantalum, niobium and a tantalum-niobium alloy.
28. The colored product according to embodiment 27, wherein the amorphous metal oxide comprises amorphous tantalum pentoxide, amorphous niobium pentoxide, amorphous titanium dioxide, and mixtures thereof.
29. The colored product according to embodiment 27 or 28, wherein the lithium-containing compound comprises lithium tantalate, lithium niobate, and mixtures thereof.
30. The colored product according to embodiment 27 or 28, wherein the colored product comprises shells, such as mobile phone shells and computer shells, etc.; accessories, such as rings, bracelets, necklaces, and watch straps; pendants such as Buddha statues, human figures, and animal images.
31. The colored product according to embodiment 30, wherein the mobile phone shell comprises a mobile phone body outer shell, a mobile phone decorative outer shell and a mobile phone protective outer shell.

### Examples

Certain exemplary embodiments are described below with reference to the accompanying drawings. As those skilled in the art would realize, the described embodiments may be modified in various different ways without departing from the spirit or scope of the present invention. Accordingly, the accompanying drawings and descriptions are considered to be illustrative in nature and not restrictive.

### Example 1

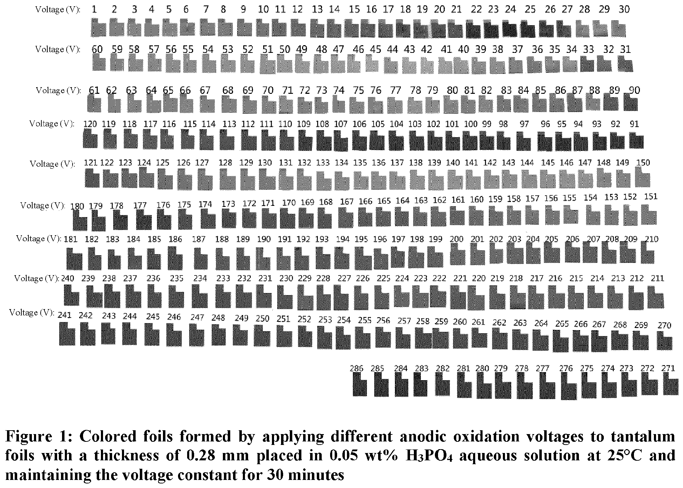

Tantalum mobile phone shells with a thickness of 0.28mm were prepared by applying an anode voltage of 1-286V respectively to the tantalum mobile phone shells placed in a 0.05 wt% aqueous H₃PO₄ solution at room temperature and maintaining the voltage constant for 40 minutes using an anodic oxidation method, to obtain colored tantalum mobile phone shells, in which a blue tantalum mobile phone shell was obtained at 30V, a golden yellow tantalum mobile phone shell was obtained at 78V, a purple tantalum mobile phone shell was obtained at 95V, a green tantalum mobile phone shell was obtained at 125V, a yellow tantalum mobile phase shell was obtained at 142V, and a rose-red tantalum mobile phase shell was obtained at 168V, as shown in Figure 1.

### Example 2

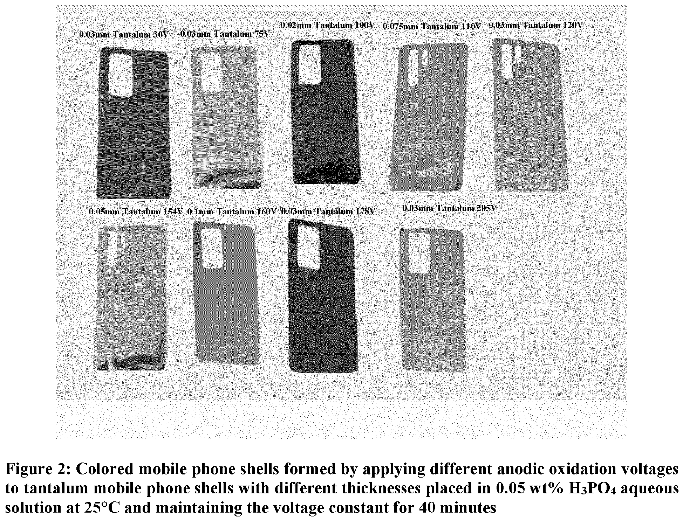

A tantalum mobile phone shells with a thickness of 0.03mm was prepared by applying an anode voltage of 30V to the tantalum mobile phone shell placed in a 0.05 wt% aqueous H₃PO₄ solution preferably at room temperature (25°C) and maintaining the voltage constant for 40 minutes using an anodic oxidation method, to obtain a blue tantalum mobile phone shell, as shown in Figure 2.

### Example 3

A tantalum mobile phone shells with a thickness of 0.03mm was prepared by applying an anode voltage of 75V to the tantalum mobile phone shell placed in a 0.05 wt% aqueous H₃PO₄ solution preferably at room temperature (25°C) and maintaining the voltage constant for 40 minutes using an anodic oxidation method, to obtain a golden yellow tantalum mobile phone shell, as shown in Figure 2.

### Example 4

A tantalum mobile phone shells with a thickness of 0.02mm was prepared by applying an anode voltage of 100V to the tantalum mobile phone shell placed in a 0.05 wt% aqueous H₃PO₄ solution preferably at room temperature (25°C) and maintaining the voltage constant for 40 minutes using an anodic oxidation method, to obtain a blue purple tantalum mobile phone shell, as shown in Figure 2.

### Example 5

A tantalum mobile phone shells with a thickness of 0.075mm was prepared by applying an anode voltage of 110V to the tantalum mobile phone shell placed in a 0.05 wt% aqueous H₃PO₄ solution preferably at room temperature (25°C) and maintaining the voltage constant for 40 minutes using an anodic oxidation method, to obtain a blue green tantalum mobile phone shell, as shown in Figure 2.

### Example 6

A tantalum mobile phone shells with a thickness of 0.03mm was prepared by applying an anode voltage of 120V to the tantalum mobile phone shell placed in a 0.05 wt% aqueous H₃PO₄ solution preferably at room temperature (25°C) and maintaining the voltage constant for 40 minutes using an anodic oxidation method, to obtain a light green tantalum mobile phone shell, as shown in Figure 2.

### Example 7

A tantalum mobile phone shells with a thickness of 0.05mm was prepared by applying an anode voltage of 154V to the tantalum mobile phone shell placed in a 0.05 wt% aqueous H₃PO₄ solution preferably at room temperature (25°C) and maintaining the voltage constant for 40 minutes using an anodic oxidation method, to obtain a light pink tantalum mobile phone shell, as shown in Figure 2.

### Example 8

A tantalum mobile phone shells with a thickness of 0.1mm was prepared by applying an anode voltage of 160V to the tantalum mobile phone shell placed in a 0.05 wt% aqueous H₃PO₄ solution preferably at room temperature (25°C) and maintaining the voltage constant for 40 minutes using an anodic oxidation method, to obtain a pink tantalum mobile phone shell, as shown in Figure 2.

### Example 9

A tantalum mobile phone shells with a thickness of 0.03mm was prepared by applying an anode voltage of 178V to the tantalum mobile phone shell placed in a 0.05 wt% aqueous H₃PO₄ solution preferably at room temperature (25°C) and maintaining the voltage constant for 40 minutes using an anodic oxidation method, to obtain a purple tantalum mobile phone shell, as shown in Figure 2.

### Example 10

A tantalum mobile phone shells with a thickness of 0.03mm was prepared by applying an anode voltage of 205V to the tantalum mobile phone shell placed in a 0.05 wt% aqueous H₃PO₄ solution preferably at room temperature (25°C) and maintaining the voltage constant for 40 minutes using an anodic oxidation method, to obtain a green tantalum mobile phone shell, as shown in Figure 2.

### Example 11

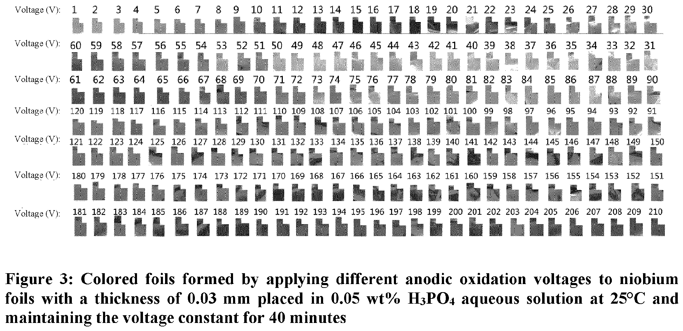

Niobium mobile phone shells with a thickness of 0.03mm were prepared by applying an anode voltage of 1-210V respectively to the niobium mobile phone shells placed in a 0.05 wt% aqueous H₃PO₄ solution at room temperature and maintaining the voltage constant for 40 minutes using an anodic oxidation method, to obtain colored niobium mobile phone shells, in which a blue niobium mobile phone shell was obtained at 25V, a silver-white niobium mobile phone shell was obtained at 36V, a golden yellow niobium mobile phone shell was obtained at 44V, a gemstone green niobium mobile phase shell was obtained at 73V, and a purplish red niobium mobile phase shell was obtained at 108V, as shown in Figure 3.

### Example 12

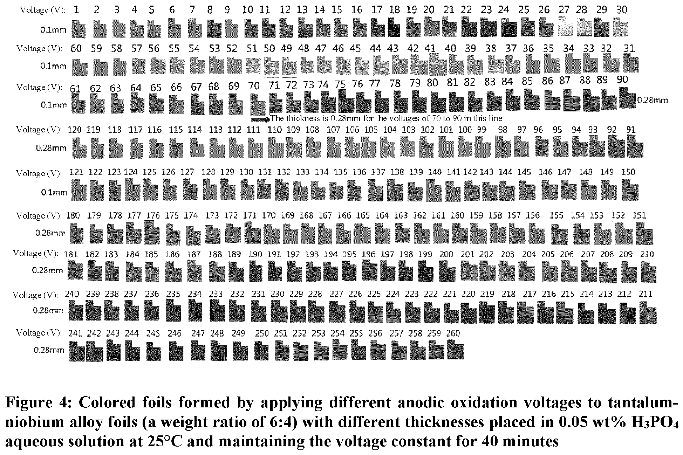

Tantalum-niobium alloy (weight ratio of 6:4) mobile phone shells with a thickness of 0.1mm or 0.28mm were prepared by applying an anode voltage of 1-260V respectively to the tantalum-niobium alloy mobile phone shells placed in a 0.05 wt% aqueous H₃PO₄ solution preferably at room temperature (25°C) and maintaining the voltage constant for 40 minutes using an anodic oxidation method, to obtain colored tantalum-niobium alloy mobile phone shells, in which a sky-blue tantalum-niobium alloy mobile phone shell was obtained at 0.1mm and 28V, a gray tantalum-niobium alloy mobile phone shell was obtained at 0.1mm and 43V, a marine green tantalum-niobium alloy mobile phone shell was obtained at 0.28mm and 100V, a violet tantalum-niobium alloy mobile phone shell was obtained at 0.1mm and 141V, and a dark brown tantalum-niobium alloy mobile phone shell was obtained at 0.28mm and 249V, as shown in Figure 4.

### Example 13

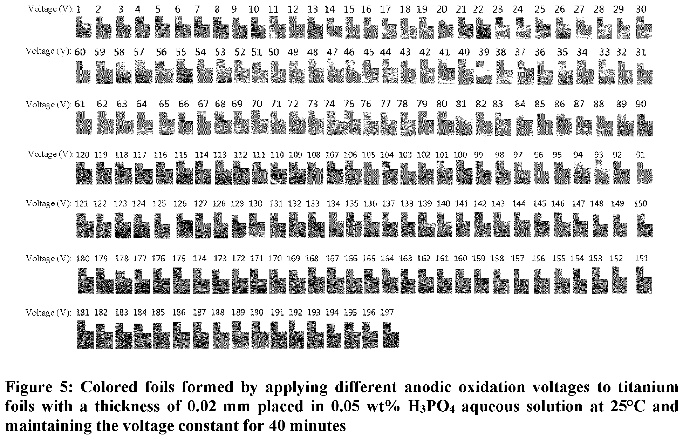

Titanium mobile phone shells with a thickness of 0.02mm were prepared by applying an anode voltage of 1-197V respectively to the titanium mobile phone shells placed in a 0.05 wt% aqueous H₃PO₄ solution preferably at room temperature (25°C) and maintaining the voltage constant for 40 minutes using an anodic oxidation method, to obtain colored titanium mobile phone shells, in which a silver-gray titanium mobile phone shell was obtained at 16V, a silver-white titanium mobile phone shell was obtained at 24V, a golden yellow titanium mobile phone shell was obtained at 82V, a rose red titanium mobile phone shell was obtained at 98V, a rose brown titanium mobile phone shell was obtained at 175V, as shown in Figure 5.

### Example 14

A titanium mobile phone shell with a thickness of 0.02mm was prepared by applying an anode voltage of 10V to the titanium mobile phone shell placed in a molten lithium nitrate solution at 300°C and maintaining the voltage constant for 2 minutes, to obtain a blue titanium mobile phone shell, as shown in Figure 6.

### Example 15

A titanium mobile phone shell with a thickness of 0.02mm was prepared by applying an anode voltage of 10V to the titanium mobile phone shell placed in a molten lithium nitrate solution at 350°C and maintaining the voltage constant for 2 minutes, to obtain a sky-blue titanium mobile phone shell, as shown in Figure 6.

### Example 16

A titanium mobile phone shell with a thickness of 0.02mm was prepared by applying an anode voltage of 12V to the titanium mobile phone shell placed in a molten lithium nitrate solution at 482°C and maintaining the voltage constant for 0.5 minute, to obtain a rose red titanium mobile phone shell, as shown in Figure 6.

### Example 17

A titanium mobile phone shell with a thickness of 0.02mm was prepared by applying an anode voltage of 12V to the titanium mobile phone shell placed in a molten lithium nitrate solution at 482°C and maintaining the voltage constant for 10 minutes, to obtain a purple titanium mobile phone shell, as shown in Figure 6.

### Example 18

A titanium mobile phone shell with a thickness of 0.02mm was prepared by applying an anode voltage of 10V to the titanium mobile phone shell placed in a molten lithium nitrate solution at 460°C and maintaining the voltage constant for 1 minute, to obtain a light yellow titanium mobile phone shell, as shown in Figure 7.

### Example 19

A titanium mobile phone shell with a thickness of 0.02mm was prepared by applying an anode voltage of 10V to the titanium mobile phone shell placed in a molten lithium nitrate solution at 460°C and maintaining the voltage constant for 2 minutes, to obtain a light yellow titanium mobile phone shell, as shown in Figure 7.

### Example 20

A titanium mobile phone shell with a thickness of 0.02mm was prepared by applying an anode voltage of 10V to the titanium mobile phone shell placed in a molten lithium nitrate solution at 460°C and maintaining the voltage constant for 5 minutes, to obtain a golden yellow titanium mobile phone shell, as shown in Figure 7.

### Example 21

A titanium mobile phone shell with a thickness of 0.02mm was prepared by applying an anode voltage of 10V to the titanium mobile phone shell placed in a molten lithium nitrate solution at 460°C and maintaining the voltage constant for 15 minutes, to obtain a dark reddish purple titanium mobile phone shell, as shown in Figure 7.

### Example 22

A titanium mobile phone shell with a thickness of 0.02mm was prepared by applying an anode voltage of 10V to the titanium mobile phone shell placed in a molten lithium nitrate solution at 460°C and maintaining the voltage constant for 20 minutes, to obtain a light purple titanium mobile phone shell, as shown in Figure 7.

### Example 23

A titanium mobile phone shell with a thickness of 0.02mm was prepared by applying an anode voltage of 10V to the titanium mobile phone shell placed in a molten lithium nitrate solution at 460°C and maintaining the voltage constant for 30 minutes, to obtain a purple titanium mobile phone shell, as shown in Figure 7.

### Example 24

A titanium mobile phone shell with a thickness of 0.02mm was prepared by applying an anode voltage of 10V to the titanium mobile phone shell placed in a molten lithium nitrate solution at 460°C and maintaining the voltage constant for 35 minutes, to obtain a maroon titanium mobile phone shell, as shown in Figure 7.

### Example 25

A titanium mobile phone shell with a thickness of 0.02mm was prepared by applying an anode voltage of 10V to the titanium mobile phone shell placed in a molten lithium nitrate solution at 460°C and maintaining the voltage constant for 60 minutes, to obtain a gemstone blue titanium mobile phone shell, as shown in Figure 7.

### Example 26

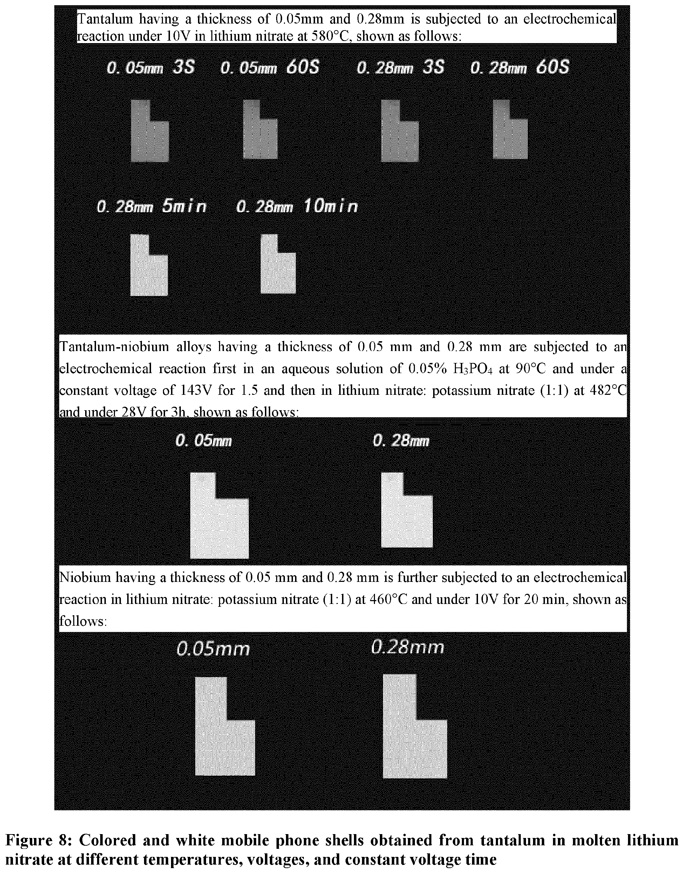

Tantalum mobile phone shells with a thickness of 0.05mm were prepared by applying an anode voltage of 10V to the tantalum mobile phone shells placed in a molten lithium nitrate solution at 580°C and maintaining the voltage constant for 3s and 60s in the reaction to obtain gray white tantalum mobile phone shells, as shown in Figure 8.

### Example 27

Tantalum mobile phone shells with a thickness of 0.28mm were prepared by applying an anode voltage of 10V to the tantalum mobile phone shells in a molten lithium nitrate solution at 580°C, and maintaining the voltage constant for 3s to 10 minutes in the reaction to obtain gray or white tantalum mobile phone shells, as shown in Figure 8.

### Example 28

A tantalum-niobium alloy mobile phone shell with a thickness of 0.05mm was prepared by firstly placing the tantalum-niobium alloy mobile phone shell in an aqueous solution of 0.05 wt% H₃PO₄ at 90°C and maintaining the voltage constant at 143V for 1.5 hours, and then applying a voltage of 28V to the tantalum-niobium alloy mobile phone shell placed in molten lithium nitrate: potassium nitrate (a weight ratio of 1:1) at 482°C and maintaining the voltage constant for 3 hours in the electrochemical reaction to obtain a white tantalum-niobium alloy phone shell, as shown in Figure 8.

### Example 29

A tantalum-niobium alloy mobile phone shell with a thickness of 0.28mm was prepared by firstly placing the tantalum-niobium alloy mobile phone shell in an aqueous solution of 0.05 wt% H₃PO₄ at 90°C and maintaining the voltage constant at 143V for 1.5 hours, and then applying a voltage of 28V to the tantalum-niobium alloy mobile phone shell placed in molten lithium nitrate: potassium nitrate (a weight ratio of 1:1) at 482°C and maintaining the voltage constant for 3 hours in the electrochemical reaction to obtain a white tantalum-niobium alloy phone shell, as shown in Figure 8.

### Example 30

A niobium mobile phone shell with a thickness of 0.05mm was prepared by applying a voltage of 10V to the niobium mobile phone shell placed in molten lithium nitrate: potassium nitrate (a weight ratio of 1:1) at 460°C and maintaining the voltage constant for 20 minutes to obtain a white niobium mobile phone shell, as shown in Figure 8.

### Example 31

A niobium mobile phone shell with a thickness of 0.28mm was prepared by applying a voltage of 10V to the niobium mobile phone shell placed in molten lithium nitrate: potassium nitrate (a weight ratio of 1:1) at 460°C and maintaining the voltage constant for 20 minutes to obtain a white niobium mobile phone shell, as shown in Figure 8.

### Example 32

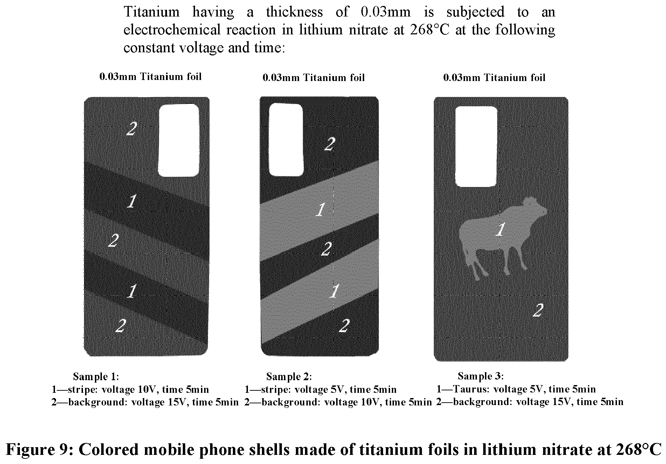

A titanium foil with a thickness of 0.03mm was prepared by applying a voltage of 15V to the titanium foil in molten lithium nitrate at 268°C under the condition of adhesive tape protection and maintaining the voltage constant for 5 minutes to obtain a blue background pattern, protecting the striped pattern with an adhesive tape, and then applying a voltage of 10V to the titanium foil in molten lithium nitrate at 268°C and maintaining the voltage constant for 5 minutes to obtain a purple striped pattern, as shown in Sample 1 in Figure 9.

### Example 33

A titanium foil with a thickness of 0.03mm was prepared by applying a voltage of 10V to the titanium foil in molten lithium nitrate at 268°C under the condition of adhesive tape protection and maintaining the voltage constant for 5 minutes to obtain a purple background pattern, protecting the striped pattern with an adhesive tape, and then applying a voltage of 5V to the titanium foil in molten lithium nitrate at 268°C and maintaining the voltage constant for 5 minutes to obtain a golden yellow striped pattern, as shown in Sample 2 in Figure 9.

### Example 34

A titanium foil with a thickness of 0.03mm was prepared by applying a voltage of 15V to the titanium foil in molten lithium nitrate at 268°C under the condition of adhesive tape protection and maintaining the voltage constant for 5 minutes to obtain a blue background pattern, protecting the Taurus pattern with an adhesive tape, and then applying a voltage of 5V to the titanium foil in molten lithium nitrate at 268°C and maintaining the voltage constant for 5 minutes to obtain a Taurus pattern, as shown in Sample 3 in Figure 9.

### Example 35

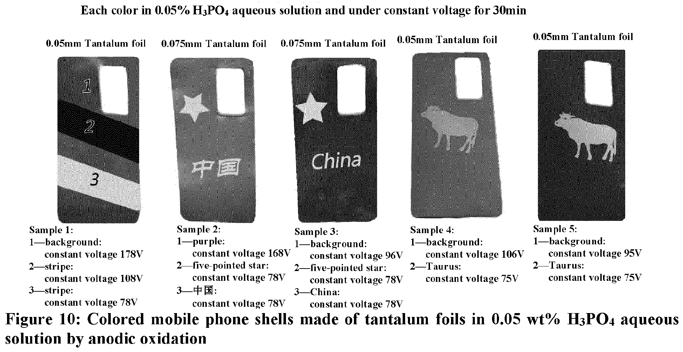

Tantalum foils with a thickness of 0.05mm or 0.075mm were prepared by applying a corresponding voltage to each color placed in 0.05 wt% H₃PO₄ aqueous solution under adhesive tape protection and in the order of the serial numbers indicated in the figure and maintaining each voltage constant for 30 minutes, giving tantalum foils each containing a variety of colored patterns, as shown in Figure 10, wherein in Sample 1, the background 1 is in purple, the stripe 2 is in blue, and the stripe 3 is in golden yellow; in Sample 2, the five-pointed star and China pattern are in golden yellow, and the background is in purple; in Sample 3, the five-pointed star and China pattern are in golden yellow, and the background is in bluish violet; in Sample 4, the Taurus pattern is in golden yellow, and the background is in blue; in Sample 5, the Taurus pattern is in golden yellow, and the background is in purple.

### Example 36

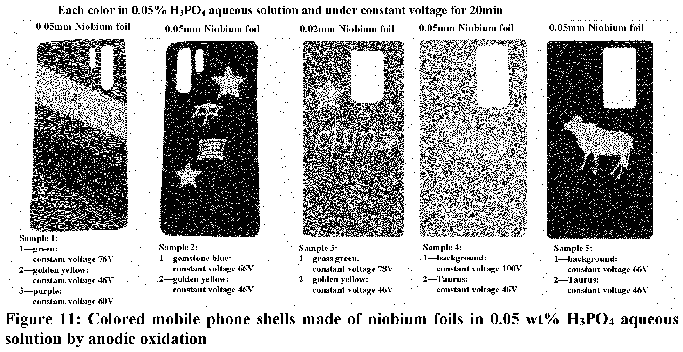

Niobium foils with a thickness of 0.05mm were prepared by applying a corresponding voltage to each color placed in 0.05 wt% H₃PO₄ aqueous solution under adhesive tape protection and in the order of the serial numbers indicated in the figure and maintaining each voltage constant for 20 minutes, giving niobium foils each containing a variety of colored patterns, as shown in Figure 11

Table 1 below shows the mobile phone radiation measured in different directions of the mobile phone when the phone is loaded with the mobile phone shell of the present invention to make a call, tested by the Electromagnetic Testing Center of Tsinghua University.

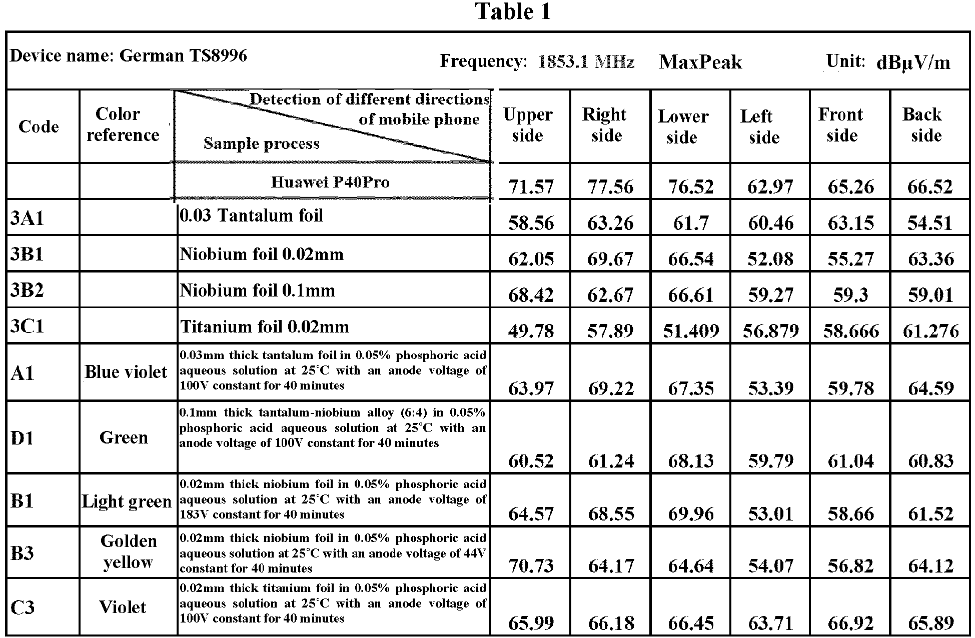

The results in Table 1 show that the mobile phone shell of the present invention has a certain radiation protection effect. Compared with the mobile phone without the mobile phone shell of the present invention, the mobile phone with the mobile phone shell of the present invention reduces the radiation amount in all directions of the mobile phone when making a call.

While particular embodiments of the present invention have been shown and described, it is to be understood that other modifications, substitutions and alternatives will occur to those skilled in the art. Such modifications, substitutions and alternative embodiments may be made without departing from the spirit and scope of the invention as determined by the appended claims. The various features of the invention are described in the appended claims.

## Claims

1. A method for manufacturing a colored product, comprising the following steps:
(1) providing a product substrate; and
(2) surface-treating the product substrate by an anodic oxidation method and/or a molten salt electrochemical method,
wherein the product substrate is made of materials selected from the group consisting of tantalum, niobium, a tantalum-niobium alloy, titanium and a titanium alloy.

2. The method according to claim 1, comprising following steps:
(1) providing a shell substrate; and
(2) surface-treating the shell substrate by an anodic oxidation method and/or a molten salt electrochemical method,
wherein the shell substrate is made of materials selected from the group consisting of tantalum, niobium, a tantalum-niobium alloy, titanium and a titanium alloy.

3. The method according to claim 2, comprising following steps:
(1) providing a mobile phone shell substrate; and
(2) surface-treating the mobile phone shell substrate by an anodic oxidation method and/or a molten salt electrochemical method,
wherein the mobile phone shell substrate is made of materials selected from the group consisting of tantalum, niobium, a tantalum-niobium alloy, titanium and a titanium alloy.

4. The method according to any one of claims 1-3, wherein step (2) comprises:
- forming a colored pattern on the substrate by an anodic oxidation method and/or a molten salt electrochemical method according to a predetermined pattern; and
- sheltering the formed colored pattern, and then forming another colored pattern by an anodic oxidation method and/or a molten salt electrochemical method.

5. The method according to any one of claims 1-3, wherein the anodic oxidation method is carried out under the following conditions: the temperature of the electrolyte solution is 20°C-600°C, the anode voltage is 1V-800V, the constant voltage time is 3 seconds-5 hours, preferably 30 seconds-1.5 hours, and the boosting current density is 1-200mA/cm², to form an amorphous metal oxide layer and/or a lithium-containing compound layer on the surface of substrates.

6. The method according to claim 5, wherein the electrolyte solution comprises an aqueous solution, a mixture of an aqueous solution and an organic compound, and a non-aqueous solution.

7. The method according to claim 6, wherein the aqueous solution comprises an aqueous solution of acid, base or salt, preferably an aqueous solution of phosphoric acid, wherein the aqueous solution of phosphoric acid has a concentration of 0.01-1 wt%, preferably 0.01-0.05 wt%.

8. The method according to claim 6, wherein in a mixture of an aqueous solution and an organic compound, the aqueous solution comprises an aqueous solution of acid, base, or salt, and the organic compound comprises an alcohol, such as ethanol, ethylene glycol, n-butanol, or any combination thereof.

9. The method according to claim 6, wherein the mixture of an aqueous solution and an organic compound comprises a mixture of a H₃PO₄ aqueous solution having a concentration of 0.01-0.05 wt% and ethylene glycol in a weight ratio of 1:1 to 3:1, preferably 1:1 to 2:1.

10. The method according to claim 6, wherein the non-aqueous solution comprises anhydrous concentrated sulfuric acid, molten salt, and a mixture of molten salt and alkaline substance.

11. The method according to claim 10, wherein the molten salt comprises molten lithium nitrate, molten sodium nitrate, molten potassium nitrate and any combination thereof.

12. The method according to claim 10, wherein the alkaline substance comprises lithium hydroxide, sodium hydroxide, potassium hydroxide and any combination thereof.

13. The method according to claim 6, wherein when the electrolyte solution is an aqueous solution or a mixture of an aqueous solution and an organic compound, the anodic oxidation method is carried out under the following conditions: the temperature of the electrolyte solution is 1-99°C, the anode voltage is 5V-600V, and the constant voltage time is within 90 minutes; preferably, the electrolyte solution temperature is from room temperature to 95°C, the anode voltage is 10-200V, and the constant voltage time is 30-60 minutes.

14. The method according to claim 13, wherein a tantalum mobile phone shell with a thickness of 0.01-0.3mm is prepared by applying an anode voltage of 72-84V to the tantalum mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.

15. The method according to claim 13, wherein a niobium mobile phone shell with a thickness of 0.01-0.3mm is prepared by applying an anode voltage of 40-48V to the niobium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.

16. The method according to claim 13, wherein a tantalum-niobium alloy mobile phone shell with a thickness of 0.01-0.3mm is prepared by applying an anode voltage of 27-28V to the tantalum-niobium alloy mobile phone shell placed in a 0.01-0.05 wt% aqueous H₃PO₄ solution preferably at room temperature (25°C) and maintaining the voltage constant for 0.5-1.5 hours using an anodic oxidation method, to obtain a sky-blue mobile phone shell.

17. The method according to claim 13, wherein a titanium mobile phone shell with a thickness of 0.01-0.3mm is prepared by applying an anode voltage of 72-84V to the titanium mobile phone shell placed in a 0.01-0.05 wt% aqueous H₃PO₄ solution preferably at room temperature (25°C) and maintaining the voltage constant for 0.5-1.5 hours using an anodic oxidation method, to obtain a golden yellow mobile phone shell.

18. The method according to claim 6, wherein when the electrolyte solution is a non-aqueous solution, the anodic oxidation method is carried out under the following conditions: the temperature of the electrolyte solution is from the melting point of the substance of the electrolyte solution to 500°C, the anode voltage is 3V-66V, and the constant voltage time is within 60 minutes; preferably, the temperature of the electrolyte solution is 250-350°C, the anode voltage is 5-40V, and the constant voltage time is 3-30 minutes.

19. The method according to claim 18, wherein the shell substrate made of titanium is placed in lithium nitrate at 460°C and subjected to an anodic oxidation method treatment at a voltage of 10V for 5 minutes to obtain a golden yellow shell.

20. The method according to any one of claims 1-3, wherein the molten salt electrochemical method comprises placing the substrate in an oxygen-containing inorganic lithium salt, a mixed melt of oxygen-containing inorganic lithium salt and lithium hydroxide, a mixed molten solution of salt and lithium hydroxide, or a mixed molten solution of lithium salt and oxygen-containing salt at 250°C-650°C, applying an anode voltage of 1-66V and maintaining the voltage constant for 0.01-60 hours, wherein the boosting current density is 1-1000 mA/cm², to obtain a white or off-white mobile phone shell.

21. The method according to claim 20, wherein the temperature of the mixed melt or the mixed molten solution is 300°C-520°C, the applied anode voltage is 5-25V, and the boosting current density is 5-20 mA/cm².

22. The method according to claim 20, wherein the oxygen-containing inorganic lithium salt is LiNO₃.

23. The method according to claim 20, wherein the shell substrate made of tantalum is placed in lithium nitrate at 440°C-650°C and subjected to a constant voltage of 8-50V for 2 minutes-5 hours to obtain a white shell.

24. The method according to claim 20, wherein the shell substrate made of niobium is placed in a lithium nitrate/potassium nitrate mixture in a weight ratio of 1:2 to 2:1, preferably a weight ratio of 1:1 at 380°C-550°C and subjected to a constant voltage of 5-28V for 2-30 minutes to obtain a white shell.

25. The method according to claim 2 or 3, comprising the following steps:
(1) providing a shell substrate; and
(2) surface-treating the shell substrate by an anodic oxidation method and a molten salt electrochemical method,
wherein the shell substrate is made of materials selected from the group consisting of tantalum, niobium and a tantalum-niobium alloy.

26. The method according to claim 25, wherein the shell substrate made of a tantalum-niobium alloy is placed in a 0.01-0.05 wt% aqueous H₃PO₄ solution and subjected to a constant voltage of 135-145V for 1-2 hours, and then placed in a lithium nitrate/potassium nitrate mixture in a weight ratio of 1:2 to 2:1, preferably a weight ratio of 1:1 at 400-585°C and subjected to a constant voltage of 25-32V for 1-3 hours to obtain a white shell.

27. A colored product manufactured according to the method of any one of claims 1-26, comprising:
(1) a product substrate; and
(2) an amorphous metal oxide layer and/or a lithium-containing compound layer, preferably an amorphous metal oxide layer, formed on the surface of the product substrate,
wherein the product substrate is made of materials selected from the group consisting of tantalum, niobium, a tantalum-niobium alloy, titanium and a titanium alloy, preferably the product substrate is made of materials selected from the group consisting of tantalum, niobium, a tantalum-niobium alloy and titanium, more preferably the product substrate is made of materials selected from the group consisting of tantalum, niobium and a tantalum-niobium alloy.

28. The colored product according to claim 27, wherein the amorphous metal oxide comprises amorphous tantalum pentoxide, amorphous niobium pentoxide, amorphous titanium dioxide, and mixtures thereof.

29. The colored product according to claim 27 or 28, wherein the lithium-containing compound comprises lithium tantalate, lithium niobate, and mixtures thereof.

30. The colored product according to claim 27 or 28, wherein the colored product comprises shells, such as mobile phone shells and computer shells; accessories, such as rings, bracelets, necklaces, and watch straps; pendants such as Buddha statues, human figures, and animal images.

31. The colored product according to claim 30, wherein the mobile phone shell comprises a mobile phone body outer shell, a mobile phone decorative outer shell and a mobile phone protective outer shell.
